(19) Europäisches Patentamt / European Patent Office / Office européen des brevets

(11) **EP 4 729 880 A2**

(12) **EUROPEAN PATENT APPLICATION**

(43) Date of publication:
22.04.2026 Bulletin 2026/17

(21) Application number: 26163246.7

(22) Date of filing: 02.08.2023

(51) International Patent Classification (IPC):
*G01B 11/00* (2006.01)

(52) Cooperative Patent Classification (CPC):
**A61N 5/1075; A61N 5/1045; G21K 1/046;**
G01B 5/207; G01B 11/02

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: 03.08.2022 CN 202222047797 U
29.12.2022 CN 202211737667
29.12.2022 CN 202223556585 U
03.07.2023 CN 202310807984

(62) Document number(s) of the earlier application(s) in
accordance with Art. 76 EPC:
**23809950.1 / 4 347 015**

(71) Applicant: **Shanghai United Imaging Healthcare**
**Co., Ltd.**
**Shanghai 201807 (CN)**

(72) Inventors:
• SONG, Bin
  **Shanghai, 201807 (CN)**
• YANG, Kun
  **Shanghai, 201807 (CN)**
• ZHANG, Jian
  **Shanghai, 201807 (CN)**

(74) Representative: **Wang, Bo**
**Panovision IP**
**Ebersberger Straße 3**
**85570 Markt Schwaben (DE)**

Remarks:
This application was filed on 09-03-2026 as a
divisional application to the application mentioned
under INID code 62.

(54) **POSITION DETECTION DEVICES OF MULTI-LEAF COLLIMATORS**

(57)     A position detection device of a multi-leaf colli-
mator (MLC) is provided. The position detection device
includes a signal emitting component, a signal feedback
component, and a processor. The signal emitting component is configured to emit a signal. The signal feedback
component is configured to receive the signal emitted by
the signal emitting component and generate a feedback
signal in response to the received signal. Installation
positions of the signal emitting component and the signal
feedback component are arranged so that the signal
emitted by the signal emitting component can be projected onto the signal feedback component. The processor communicatively connected with the signal feedback
component and configured to receive the feedback signal
from the signal feedback component and determine a
moving stroke of a leaf of the MLC based on the feedback
signal.

FIG. 3

**Description**

**CROSS-REFERENCE TO RELATED APPLICATIONS**

This application claims priority to Chinese Patent Application No. 202222047797.3 filed on August 3, 2022, Chinese Patent Application No. 202211737667.0 filed on December 29, 2022, Chinese Patent Application No. 202223556585.4 filed on December 29, 2022, and Chinese Patent Application No. 202310807984.3 filed on July 3, 2023, the entire contents of each of which are hereby incorporated by reference.

**TECHNICAL FIELD**

The present disclosure generally relates to medical devices, and more particularly, relates to a position detection device of a multi-leaf collimator (MLC) of a radiotherapy device, and a method for determining a position of each leaf of the MLC.

**BACKGROUND**

Radiotherapy has been widely employed in cancer therapy by emitting ionizing radiation towards a tumor. In radiotherapy, it is desired that the tumor receives sufficient radiation, while the damage to an organ at risk (OAR) is minimized as much as possible. A multi-leaf collimator (MLC) is commonly used in radiotherapy to shape the radiation to the tumor. For instance, by moving leaves of the MLC to their respective desired positions, various radiation fields may be formed to conform to the shape of the tumor. That is, in order to ensure the radiation source delivers correctly shaped radiation, the position of each leaf needs to be accurately determined. Thus, it is desirable to provide a position detection device of the MLC to determine a position of each leaf of the MLC.

**SUMMARY**

An aspect of the present disclosure relates to a position detection device of a multi-leaf collimator (MLC). The position detection device includes a signal emitting component, a signal feedback component, and a processor. The signal emitting component is configured to emit a signal. The signal feedback component is configured to receive the signal emitted by the signal emitting component and generate a feedback signal in response to the received signal. Installation positions of the signal emitting component and the signal feedback component are arranged so that the signal emitted by the signal emitting component can be projected onto the signal feedback component. The processor communicatively connected with the signal feedback component and configured to receive the feedback signal from the signal feedback component and determine a moving stroke of a leaf of the MLC based on the feedback signal.

Another aspect of the present disclosure relates to a position detection method of a multi-leaf collimator (MLC). The position detection method includes emitting a signal by a signal emitting component; receiving, by a signal feedback component, the signal emitted by the signal emitting component and generate, by the signal feedback component, a feedback signal in response to the received signal, wherein installation positions of the signal emitting component and the signal feedback component are arranged so that the signal emitted by the signal emitting component can be projected onto the signal feedback component; receiving, by a processor, the feedback signal from the signal feedback component, the processor being communicatively connected with the signal feedback component; and determining, by the processor, a moving stroke of a leaf of the MLC based on the feedback signal.

A further aspect of the present disclosure relates to a multi-leaf collimator (MLC) including a position detection device. The position detection device includes a signal emitting component, a signal feedback component, and a processor. The signal emitting component is configured to emit a signal. The signal feedback component is configured to receive the signal emitted by the signal emitting component and generate a feedback signal in response to the received signal. Installation positions of the signal emitting component and the signal feedback component are arranged so that the signal emitted by the signal emitting component can be projected onto the signal feedback component. The processor communicatively connected with the signal feedback component and configured to receive the feedback signal from the signal feedback component and determine a moving stroke of a leaf of the MLC based on the feedback signal.

A still further aspect of the present disclosure relates to a radiotherapy device including a multi-leaf collimator (MLC). The MLC includes a position detection device. The position detection device includes a signal emitting component, a signal feedback component, and a processor. The signal emitting component is configured to emit a signal. The signal feedback component is configured to receive the signal emitted by the signal emitting component and generate a feedback signal in response to the received signal. Installation positions of the signal emitting component and the signal feedback component are arranged so that the signal emitted by the signal emitting component can be projected onto the signal feedback component. The processor communicatively connected with the signal feedback component and configured to receive the feedback signal from the signal feedback component and determine a moving stroke of a leaf of the MLC based on the feedback signal.

A still further aspect of the present disclosure relates to an image-guided radiotherapy (IGRT) including an imaging device and a radiotherapy device. The imaging device is configured to determine a target area of a subject to be treated and/or guide an emission of treatment radiations. The radiotherapy device is configured to deliver the treatment radiations to the target area. An isocenter of the radiotherapy device coincides with an isocenter of the imaging device. The radiotherapy device includes a multi-leaf collimator (MLC). The MLC includes a position detection device. The position detection device includes a signal emitting component, a signal feedback component, and a processor. The signal emitting component is configured to emit a signal. The signal feedback component is configured to receive the signal emitted by the signal emitting component and generate a feedback signal in response to the received signal. Installation positions of the signal emitting component and the signal feedback component are arranged so that the signal emitted by the signal emitting component can be projected onto the signal feedback component. The processor communicatively connected with the signal feedback component and configured to receive the feedback signal from the signal feedback component and determine a moving stroke of a leaf of the MLC based on the feedback signal.

Additional features may be set forth in part in the description which follows, and in part may become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

## BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:

FIG. 1 is a schematic diagram illustrating an exemplary radiotherapy system according to some embodiments of the present disclosure;

FIG. 2A-2B are schematic diagrams illustrating a top view of an exemplary MLC according to some embodiments of the present disclosure;

FIG. 3 is a schematic diagram illustrating an exemplary position detection device of a leaf of an MLC according to some embodiments of the present disclosure;

FIG. 4 is a schematic diagram illustrating exemplary installation positions of a signal emitting component and a signal feedback component according to some embodiments of the present disclosure;

FIG. 5 is a schematic diagram illustrating exemplary installation positions of a signal emitting component and a signal feedback component according to some embodiments of the present disclosure;

FIG. 6 is a schematic diagram illustrating exemplary installation positions of a signal emitting component, an emitting element, and a reflecting element according to some embodiments of the present disclosure;

FIG. 7 is a schematic diagram illustrating exemplary installation positions of a signal emitting component, an emitting element, and a reflecting element according to some embodiments of the present disclosure;

FIG. 8 is a schematic diagram illustrating an exemplary signal feedback component according to some embodiments of the present disclosure;

FIG. 9A is a schematic diagram illustrating an exemplary position detection device of a leaf of an MLC according to some embodiments of the present disclosure;

FIG. 9B is a schematic diagram illustrating exemplary installation positions of fiber optic probes of position detection devices of leaves of an MLC according to some embodiments of the present disclosure;

FIG. 10 is a schematic diagram illustrating an exemplary process of determining a moving stroke of a leaf of an MLC according to some embodiments of the present disclosure;

FIG. 11 is a schematic diagram illustrating an exemplary position detection device of a leaf of an MLC according to some embodiments of the present disclosure;

FIG. 12 is a schematic diagram illustrating an exemplary position detection device of a leaf of an MLC according to some embodiments of the present disclosure;

FIG. 13 is a schematic diagram illustrating an exemplary position detection device of a leaf of an MLC according to some embodiments of the present disclosure;

FIG. 14 is a schematic diagram illustrating an exemplary position detection device of a leaf of an MLC according to some embodiments of the present disclosure;

FIG. 15 is a schematic diagram illustrating an exemplary position detection device of a leaf of an MLC according to some embodiments of the present disclosure; and

FIG. 16 is a flowchart illustrating an exemplary process for determining a position of a leaf of the MLC according to some embodiments of the present disclosure.

## DETAILED DESCRIPTION

In the following detailed description, numerous specific details may be set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high-level, without detail, in order to avoid unnecessarily obscuring aspects of the present disclosure. Various modifications to the disclosed embodiments may be readily apparent to those skilled in the art, and the general principles defined herein may be applied to other embodiments and applications without departing from the spirit and scope of the present disclosure. Thus, the present disclosure may be not limited to the embodiments shown, but to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

Certain terminology has been used to describe embodiments of the present disclosure. For example, the terms "one embodiment," "an embodiment," and/or "some embodiments" may mean that a particular feature, structure or characteristic described in connection with the embodiment is in at least one embodiment of the present disclosure. Therefore, it is emphasized and should be appreciated that two or more references to "an embodiment" or "one embodiment" or "an alternative embodiment" in various portions of this specification may not necessarily all referring to the same embodiment. Furthermore, the particular features, structures or characteristics may be combined as suitable in one or more embodiments of the present disclosure.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings may be for the purpose of illustration and description only and may be not intended to limit the scope of the present disclosure.

The flowcharts used in the present disclosure may illustrate operations that systems implement according to some embodiments of the present disclosure. It is to be expressly understood, the operations of the flowcharts may be implemented not in order. Conversely, the operations may be implemented in inverted order, or simultaneously. Moreover, one or more other operations may be added to the flowcharts. One or more operations may be removed from the flowcharts.

Generally, leaf position detection devices of an MLC can be classified into a contact leaf position detection device and a non-contact leaf position detection device. The contact leaf position detection device includes a thin film potentiometer. The thin film potentiometer often causes device wear during the leaf position detection process, and has defects such as short service life, poor position detection reliability, and insufficient position detection accuracy. The contact leaf position detection device includes a ruby CCD (Charge Coupled Device) camera and a magnetic grid detection device. The long-term exposure of the CCD camera to the radiation environment (e.g., an image-guided radiotherapy (IGRT) system) makes the CCD camera prone to have failure, so the CCD camera needs to be replaced frequently, and the CCD camera is only suitable for a single-layer MLC. In addition, the magnetic grid detection device can only be used in a nonmagnetic environment, which limits the application of the contact leaf position detection device.

The present disclosure provides a position detection device of an MLC. The position detection device may include a signal emitting component, a signal feedback component, and a processor. The signal emitting component may be configured to emit a signal. The signal feedback component may be configured to receive the signal emitted by the signal emitting component and generate a feedback signal in response to the received signal. Installation positions of the signal emitting component and the signal feedback component may be arranged so that the signal emitted by the signal emitting component can be projected onto the signal feedback component. The processor may be communicatively connected with the signal feedback component and configured to receive the feedback signal from the signal feedback component and determine a moving stroke of a leaf of the MLC based on the feedback signal. The position detection device of the MLC in the present disclosure can automatically, accurately, and reliably determine the moving stroke of the leaf of the MLC in a magnetic environment (e.g., the IGRT system).

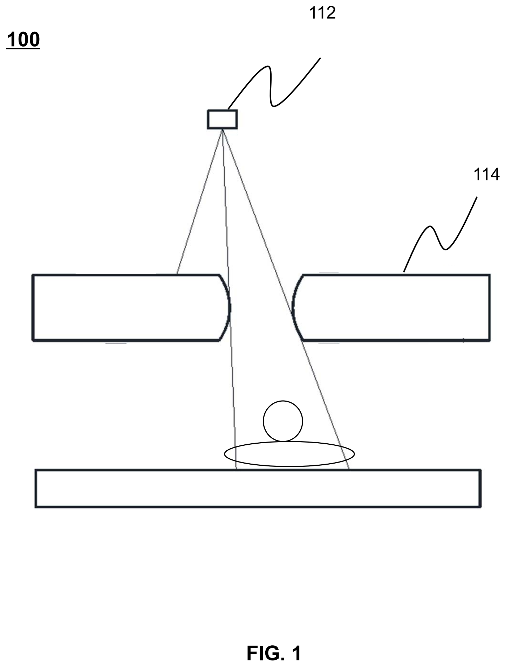

FIG. 1 is a schematic diagram illustrating an exemplary radiotherapy device 100 according to some embodiments of the present disclosure. The radiotherapy device 100 may perform radiotherapy treatment on at least part of a subject.

The subject may be biological or non-biological. For example, the subject may include a patient, a man-made object, etc. As another example, the subject may include a specific portion, organ, and/or tissue of the patient. For example, the subject may include head, neck, thorax, cardiac, stomach, blood vessel, soft tissue, tumor, nodules, or the like, or a combination thereof. In some embodiments, the subject may include a region of interest (ROI), such as a tumor, a node, etc. In some embodiments, the radiotherapy device 100 may include a single modality apparatus, for example, an X-ray therapy apparatus, a Co-60 teletherapy apparatus, a medical electron accelerator, etc. In some embodiments, the radiotherapy device 100 may be a multi-modality (e.g., two-modality) apparatus to acquire a medical image relating to the at least part of the subject and perform radiotherapy treatment on the at least part of the subject. Merely by way of example, the multi-modality apparatus may include an image-guided radiotherapy (IGRT) system, for example, a CT guided radiotherapy system, an MRI guided radiotherapy system, etc. The IGRT may include an imaging device and the radiotherapy device 100. The imaging device may be configured to determine a target area of a subject to be treated and/or guide an emission of treatment radiations. The radiotherapy device 100 may be configured to perform the radiotherapy treatment on the target area by delivering the treatment radiations to the target area. An isocenter of the radiotherapy device 100 may coincide with an isocenter of the imaging device. Merely by way of example, the imaging device includes a computed tomography (CT) device, a magnetic resonance imaging (MRI) device, a positron emission tomography (PET) device, an ultrasonic imaging diagnostic device, a single photon emission computed tomography device, an X-ray imaging device, or the like, or a combination thereof.

In some embodiments, the radiotherapy device 100 may include a gantry and a treatment head, wherein the treatment head may be mechanically connected to the gantry. The treatment head may include a radiation source 112 and a multi-leaf collimator (MLC) 114. The radiation source 112 may emit radiation beams to the subject. The MLC 114 may be configured to collimate radiation beams emitted from the radiation source 112 and/or define the beam shape(s) thereof to form one or more radiation fields. An MLC may include multiple leaves. The multiple leaves may be driven by one or more driving mechanisms (e.g., motors) to move to specific positions to expand or narrow the one or more radiation fields.

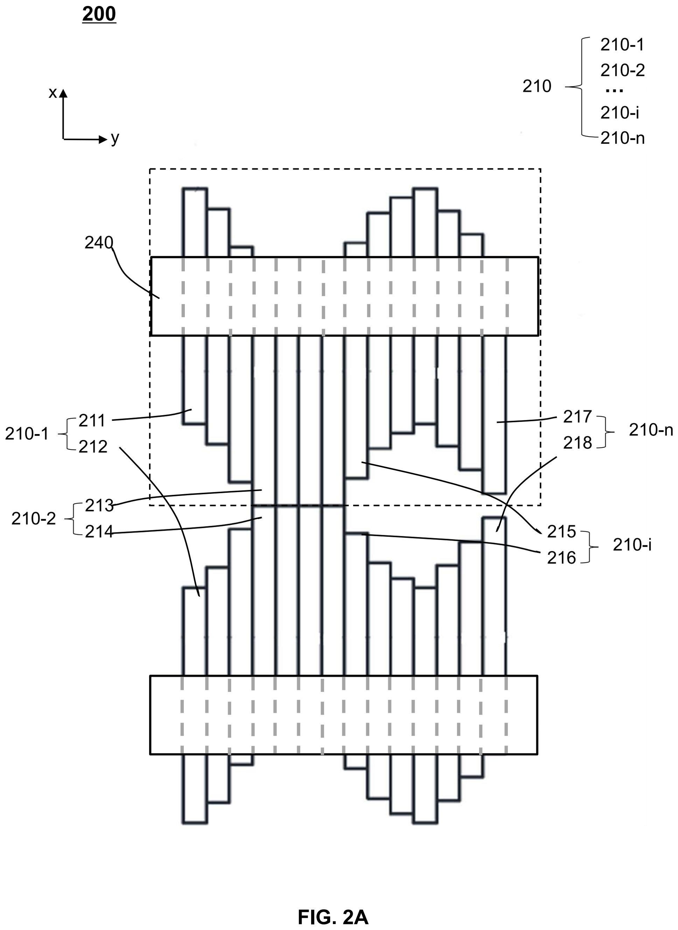

FIG. 2A-2B are schematic diagrams illustrating a top view of an exemplary MLC according to some embodiments of the present disclosure. As shown in FIG. 2A, the MLC 200 may include multiple leaf pairs 210 (e.g., a leaf pair 210-1, a leaf pair 210-2, a leaf pair 210-i, ..., a leaf pair 210-n, etc.). Each of the multiple leaf pairs 210 may include two leaves arranged oppositely. For example, the leaf pair 210-1 may include a leaf 211 and a leaf 212; the leaf pair 210-2 may include a leaf 213 and a leaf 214; the leaf pair 210-i may include a leaf 215 and a leaf 216; and the leaf pair 210-n may include a leaf 217 and a leaf 218.

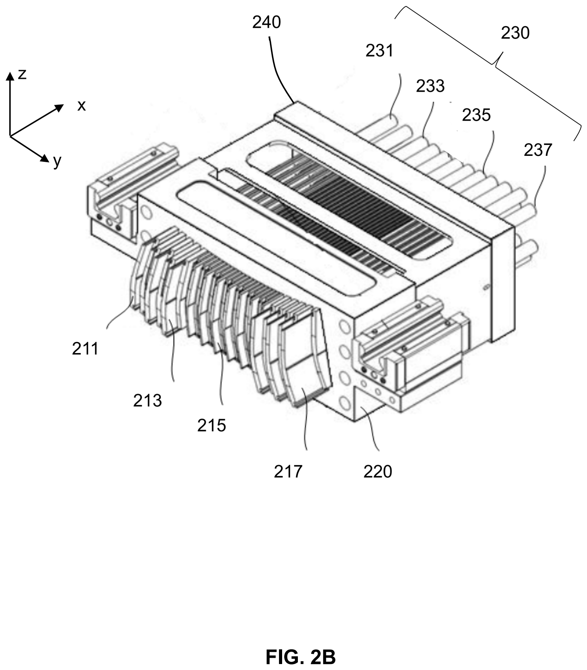

FIG. 2B illustrates a perspective view of a portion of the MLC 200 in a dashed box in the FIG. 2A. As shown in FIG. 2B, the MLC 200 may further include a guide rail box 220, multiple driving mechanisms (e.g., motors) 230 (e.g., a motor 231, a motor 233, a motor 235, a motor 237, etc.), and a mounting seat 240. The guide rail box 220 may include multiple guide rails. Each guide rail of the multiple guide rails may be configured to guide a movement of a leaf of the multiple leaf pairs 210. The multiple driving mechanisms 230 may be supported by and/or accommodated within the mounting seat 240. The multiple driving mechanisms 230 may be configured to actuate the leaves of the multiple leaf pairs 210 to move along the multiple guide rails. Each driving mechanism 230 may be configured to actuate one or more of the leaves. The leaves of the multiple leaf pairs 210 may be configured to shield a portion of radiation beams emitted by a radiation source (e.g., the radiation source 112 illustrated in FIG. 1).

In some embodiments, at least some of the leaves of the multiple leaf pairs 210 may be actuated and moved simultaneously. By simultaneously actuating and moving at least some of the leaves of the multiple leaf pairs 210, an aperture (also referred to as a radiation field) may be formed. A portion of radiation beams emitted from the radiation source may pass through the aperture, and further be delivered to a treatment region (e.g., a tumor) of a subject. The other portion of radiation beams may be blocked by the leaves of the MLC 200, which may prevent healthy tissues and/or organs around the treatment region from being irradiated by the radiation beams, and accordingly, the precise delivery of radiation to the treatment region may be achieved. In some embodiments, the multiple driving mechanisms 230 may facilitate the movement of the leaves of the multiple leaf pairs 210 such that the MLC 200 can define different apertures (e.g., change a first aperture shape to a second aperture shape). For example, a leaf 211 may be actuated, by a corresponding driving mechanism 231, to move from a first position (e.g., the current position) to a second position (e.g., a desired position).

In some embodiments, a moving direction of the leaves of the MLC 200 may be defined as a first direction (i.e., an x-axis direction illustrated in FIGs. 2A and 2B), an arrangement direction of the leaves of the MLC 200 may be defined as a second direction (i.e., a y-axis direction illustrated in FIGs. 2A and 2B), and a direction (e.g., a direction along the radiation beams) perpendicular to the first direction and the second direction may be defined as a third direction (i.e., a z-axis direction illustrated in FIG. 2B).

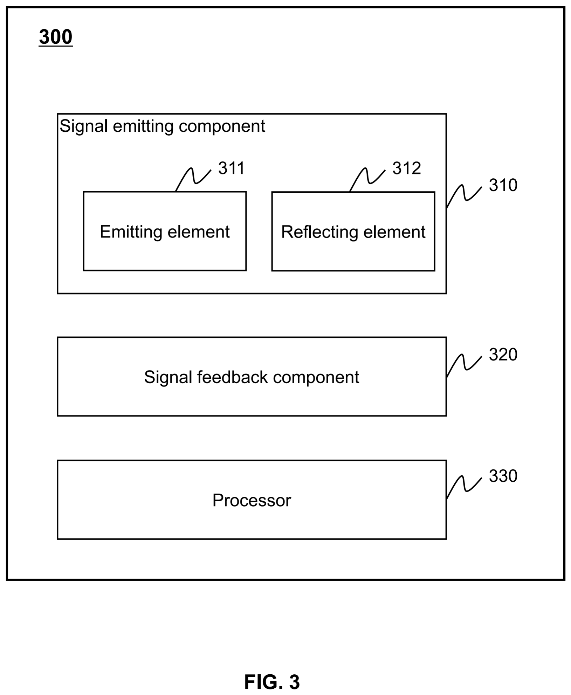

In some embodiments, the MLC 200 may include multiple position detection devices (not shown in FIG. 2A-2B). Each of the multiple position detection devices may correspond to a leaf of the MLC 200 and be configured to determine a moving stroke (e.g., from a history position to a current position) of the leaf of the MLC 200 and further determine the current position of the leaf of the MLC 200 based on the history position and the moving stroke of the leaf of the MLC 200. More descriptions of the position detection device may be found elsewhere in the present disclosure (e.g., FIG. 3 and the descriptions thereof).

FIG. 3 is a schematic diagram illustrating an exemplary position detection device of a leaf of an MLC according to some embodiments of the present disclosure. As shown in FIG. 3, the position detection device 300 may include a signal emitting component 310, a signal feedback component 320, and a processor 330. It should be noted that the position detection device 300 may be an exemplary embodiment of the position detection device described in FIGs. 2A-2B.

The signal emitting component 310 may be configured to emit a signal. The signal may be a pulse signal with directional, energy-concentrating properties. Merely by way of example, the signal includes an optical signal (e.g., a light beam) or a laser signal, and accordingly, the signal emitting component 310 is a light-emitting diode or a laser emitter.

In some embodiments, as shown in FIG. 3, the signal emitting component 310 includes an emitting element 311 and a reflecting element 312. The emitting element 311 may be configured to emit the signal. Merely by way of example, the emitting element 311 is a light-emitting diode or a laser emitter. The reflecting element 312 may be configured to reflect the signal emitted by the emitting element 311 to the signal feedback component 320. In some embodiments, the reflecting element 312 may include reflective material. Merely by way of example, the reflecting element 312 is a reflector that can reflect optical light or laser signals.

The signal feedback component 320 may be configured to receive the signal emitted by the signal emitting component 310 and generate a feedback signal in response to the received signal. In response to the received signal, the signal feedback component 320 may convert the received signal into the feedback signal. Merely by way of example, the feedback signal includes a current signal, a digital signal, a clock signal, a reflected signal of the received signal, or the like, or any combination thereof. In some embodiments, the signal feedback component 320 includes a PIN photoresistor. The PIN photoresistor refers to a resistor made of the photoelectric effect of a semiconductor, and resistance value of the PIN photoresistor changes with the intensity of the incident light.

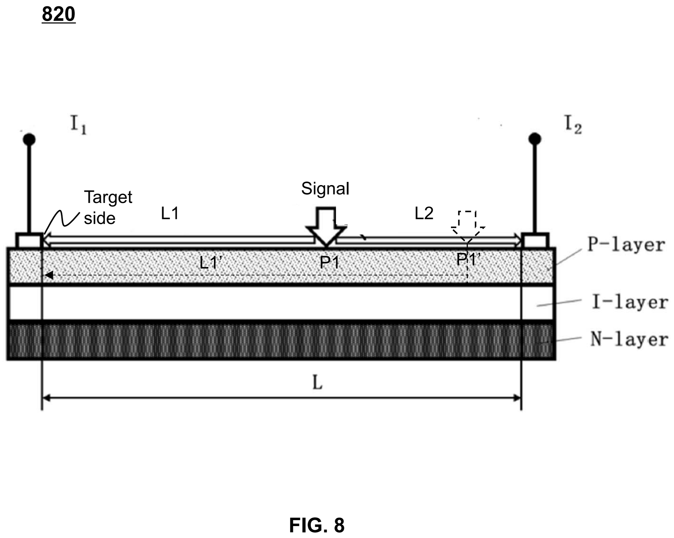

The processor 330 may be communicatively connected with the signal feedback component 320 via, for example, a wireless connection, a wired connection, or a combination thereof. The processor 330 may execute computer instructions (e.g., program code) and perform functions described herein. The computer instructions may include, for example, routines, programs, objects, components, data structures, procedures, modules, and functions, which perform particular functions described herein. Specifically, the processor 330 may be configured to receive the feedback signal from the signal feedback component and determine a moving stroke of a leaf of the MLC based on the feedback signal. More descriptions of the determination of the moving stroke of the leaf of the MLC may be found elsewhere in the present disclosure (e.g., FIG. 8 and the descriptions thereof).

In some embodiments, the processor 330 may include one or more hardware processors, such as a microcontroller, a microprocessor, a reduced instruction set computer (RISC), an application specific integrated circuits (ASICs), an application-specific instruction-set processor (ASIP), a central processing unit (CPU), a graphics processing unit (GPU), a physics processing unit (PPU), a microcontroller unit, a digital signal processor (DSP), a field programmable gate array (FPGA), an advanced RISC machine (ARM), a programmable logic device (PLD), any circuit or processor capable of executing one or more functions, or the like, or a combinations thereof.

Installation positions of the signal emitting component 310 and the signal feedback component 320 may be arranged so that the signal emitted by the signal emitting component 310 can be projected onto the signal feedback component 320. In some embodiments, the signal feedback component 320 and the signal emitting component 310 are arranged on the same side of the leaf of the MLC, and the signal feedback component 320 or the signal emitting component 310 is mounted on the leaf of the MLC.

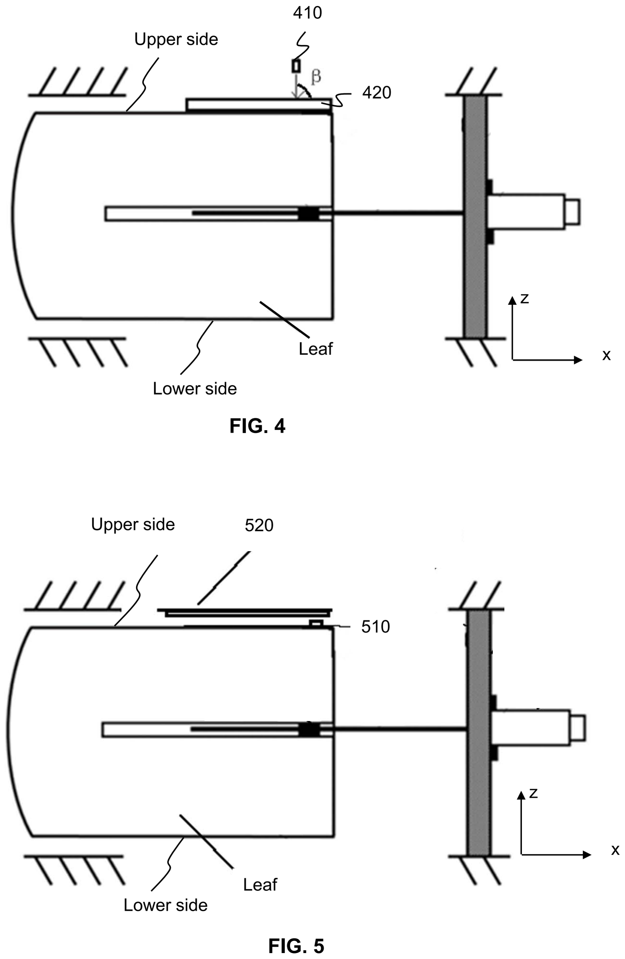

For example, FIG. 4 is a schematic diagram illustrating exemplary installation positions of a signal emitting component and a signal feedback component according to some embodiments of the present disclosure. As shown in FIG. 4, a signal feedback component 420 is arranged on an upper side of a leaf of an MLC. A signal emitting component 410 is arranged at a position above the signal feedback component 420 along the third direction (i.e., the z-axis direction) and be at a preset distance from the signal feedback component 420, so that the signal emitted by the signal emitting component 410 can be quickly received by the signal feedback component 420, thereby reducing the signal loss in the signal transmission process and improving the accuracy of the determined moving stroke of the leaf of the MLC.

Merely by way of example, the signal emitting component 410 is arranged in a box configured to accommodate the MLC and on the top of the box. The above arrangement of the signal emitting component 410 may avoid the problem that wires of the signal emitting component 410 are entangled and affect the use of the signal emitting component 410 when the leaf moves. The signal emitted by the signal emitting component 410 is received by the signal feedback component 420 at a preset angle $\beta$. The preset angle $\beta$ may be larger than 0 degrees and less than 180 degrees. For example, the preset angle $\beta$ is 45 degrees, 75 degrees, 90 degrees ,135 degrees, or 165 degrees. The preset angle $\beta$ may be adjusted by adjusting the installation position of the signal emitting component 410 and/or the signal feedback component 420. It should be noted that the signal emitting component 410 and the signal feedback component 420 may be exemplary embodiments of the signal emitting component 310 and the signal feedback component 320 illustrated in FIG. 3, respectively.

As another example, FIG. 5 is a schematic diagram illustrating exemplary installation positions of a signal emitting component and a signal feedback component according to some embodiments of the present disclosure. As shown in FIG. 5, a signal emitting component 510 is arranged on an upper side of a leaf of an MLC. A signal feedback component 520 is arranged at a position above the signal emitting component 510 along the third direction (i.e., the z-axis direction) and be at a preset distance from the signal emitting component 510, so that the signal emitted by the signal emitting component 510 can be quickly received by the signal feedback component 520. Merely by way of example, the signal feedback component 520 is arranged in the box configured to accommodate the MLC and on the top of the box. It should be noted that the signal emitting component 510 and the signal feedback component 520 may be exemplary embodiments of the signal emitting component 310 and the signal feedback component 320 illustrated in FIG. 3, respectively.

It should be noted that the above arrangement manners of the signal emitting component and the signal feedback component in FIG. 4 and FIG. 5 are merely provided for illustration purposes, and not intended to limit the scope of the present disclosure. In some embodiments, the signal feedback component and the signal emitting component are arranged at other positions. For example, the signal feedback component is arranged on a lower side of the leaf of the MLC, and the signal emitting component is arranged at a position below the signal feedback component along the third direction and be at a preset distance from the signal feedback component. As another example, the signal emitting component is arranged on the lower side of the leaf of the MLC, and the signal feedback component is arranged at the position below the signal emitting component along the third direction and be at a preset distance from the signal emitting component.

As described in connection with FIG. 3, in some embodiments, the signal emitting component 310 includes an emitting element 311 configured to emit the signal and a reflecting element 312 configured to reflect the signal emitted by the emitting element 311 to the signal feedback component 320. An installation position of the reflecting element 312 is arranged so that the signal emitted by the emitting element 311 can be projected onto the reflecting element 312 and further projected to the signal feedback component 320 through the reflecting element 312.

In some embodiments, the reflecting element 312 is arranged on the leaf of the MLC and moves with the leaf along the first direction (i.e., the x-axis direction). The emitting element 311 is arranged on a mounting seat of a driving mechanism of the leaf of the MLC, and the signal feedback component 320 is arranged above or below the leaf of the MLC along the third direction. Accordingly, the reflecting element 312 moves relative to the signal feedback component 320 in the first direction.

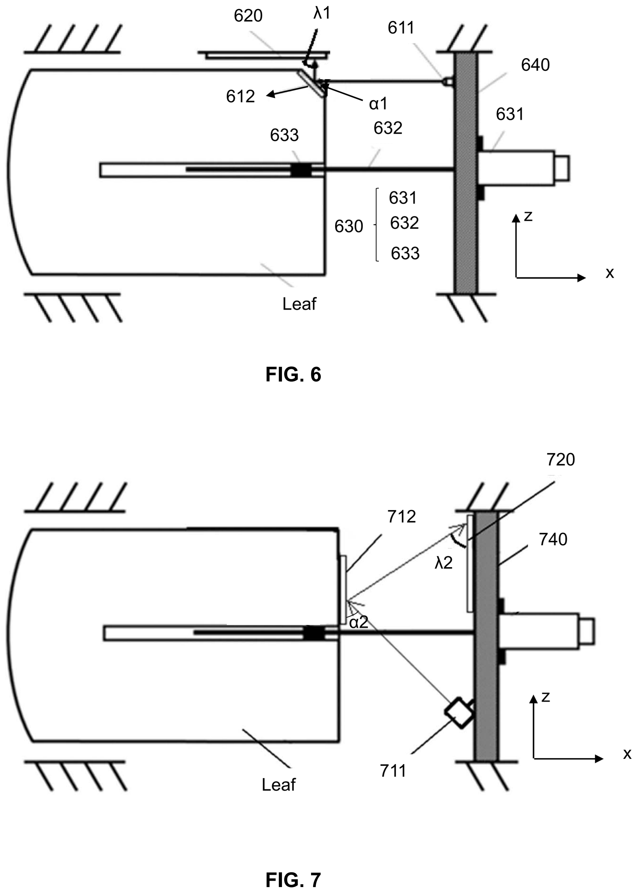

FIG. 6 is a schematic diagram illustrating exemplary installation positions of a signal emitting component, an emitting element, and a reflecting element according to some embodiments of the present disclosure. As shown in FIG. 6, a reflecting element 612 is obliquely arranged at an upper corner of a leaf of an MLC close to a mounting seat 640 of a driving mechanism 630 of the leaf of the MLC. The signal emitted by an emitting element 611 can be projected onto the reflecting element 612 and further projected to the signal feedback component 620, an inclination angle of the reflecting element 612 relative to a vertical plane where the second direction and the third direction are located may be any angle. For example, the inclination angle is 35 degrees, 45 degrees, or 55 degrees. The mounting seat 640 may be configured to support a driving mechanism 630 of the leaf of the MLC. The driving mechanism 630 may include a motor 631, a screw rod 632, and a nut 633. The driving mechanism 630 may be configured to drive the screw rod 632 to move. The nut 633 may be mounted on the leaf of an MLC. The screw rod 632 may cooperate with the nut 633 to actuate the leaf to move.

The emitting element 611 is arranged on the mounting seat 640 of the driving mechanism 630 of the leaf of the MLC. The signal emitted by the emitting element 611 may be parallel to or at an angle with respect to the first direction (i.e., the x-axis direction). The angle may be larger than and equal to 0 degrees and less than 90 degrees. The signal emitted by the emitting element 611 can be projected onto the reflecting element 612 at a second preset angle $\alpha 1$. The second preset angle $\alpha 1$ may be larger than 0 degrees and less than 90 degrees. For example, the second preset angle $\alpha 1$ is 35 degrees, 55 degrees, or 75 degrees. The second preset angle $\alpha 1$ may be adjusted by adjusting the installation positions of the emitting element 611 and/or the reflecting element 612. As long as the signal emitted by the emitting element 611 can be projected onto the reflecting element 612, the emitting element 611 may be mounted on any position on the mounting seat 640 of the driving mechanism 630 of the leaf of the MLC.

The signal feedback component 620 is arranged above the leaf of the MLC along the third direction. The signal reflected by the reflecting element 612 can be further projected to the signal feedback component 620 at a third preset angle $\lambda 1$. The third preset angle $\lambda 1$ may be larger than 0 degrees and less than 180 degrees. For example, the third preset angle $\lambda 1$ is 45 degrees, 75 degrees, 90 degrees, 135 degrees, or 165 degrees. The third preset angle $\lambda 1$ may be adjusted by adjusting the installation positions of the reflecting element 612 and/or the signal feedback component 620. As long as the signal feedback component 620 can receive the signal reflected by the reflecting element 612, the position of the signal feedback component 620 may be adjusted arbitrarily.

It should be noted that the signal feedback component 620, the emitting element 611, and the reflecting element 612 may be exemplary embodiments of the signal feedback component 320, the emitting element 311, and the reflecting element 312 illustrated in FIG.3, respectively. The driving mechanism 630 and the mounting seat 640 may be exemplary embodiments of the driving mechanism 230 and the mounting seat 240 illustrated in FIGs. 2A-2B, respectively.

It should be noted that the above arrangement manners of the signal feedback component 620, the emitting element 611, and the reflecting element 612 in FIG. 6 are merely provided for illustration purposes, and not intended to limit the scope of the present disclosure. In some embodiments, the signal feedback component 620, the emitting element 611, and the reflecting element 612 are arranged at other positions. For example, the reflecting element 612 is obliquely arranged at a lower corner of the leaf of an MLC close to the mounting seat 640, the emitting element 611 is arranged on the mounting seat 640 of the driving mechanism 630 of the leaf of the MLC, and the signal feedback component 620 is arranged below the leaf of the MLC along the third direction.

In some embodiments, the emitting element 311 and the signal feedback component 320 are arranged on the mounting seat of the driving mechanism of the leaf of the MLC.

FIG. 7 is a schematic diagram illustrating exemplary installation positions of a signal emitting component, an emitting element, and a reflecting element according to some embodiments of the present disclosure. As shown in FIG. 7, a reflecting element 712 is arranged on a side of a leaf of an MLC close to a mounting seat 740. As long as the signal emitted by an emitting element 711 can be projected onto the reflecting element 712 and further projected to a signal feedback component 720, the reflecting element 712 can be mounted at any position on the side of the leaf of the MLC close to the mounting seat 740. In some embodiments, the reflecting element 712 is arranged in parallel to the side of the leaf close to the mounting seat 740. In some embodiments, the reflecting element 712 is arranged obliquely relative to the side of the leaf close to the mounting seat 740. An inclination angle of the reflecting element 612 relative to the side of the leaf close to the mounting seat 740 may be in a range of -10-10 degrees.

The emitting element 711 and the signal feedback component 720 are arranged on the mounting seat 740. As long as the signal emitted by the emitting element 711 can be projected onto the reflecting element 712 and further projected to the signal feedback component 720, the installation positions of the emitting element 711 and the signal feedback component 720 may be adjusted arbitrarily. For example, as shown in FIG. 7, the signal feedback component 720 is mounted above the emitting element 711 along the third direction.

In some embodiments, the signal emitted by the emitting element 711 may form an angle with the first direction (i.e., the x-axis direction). The angle may be larger than 0 degrees and less than 90 degrees. The signal emitted by the emitting element 711 can be projected onto the reflecting element 712 at a fourth preset angle $\alpha2$. The fourth preset angle $\alpha2$ may be larger than 0 and less than 180 degrees. For example, the fourth preset angle $\alpha2$ is 45 degrees, 75 degrees, 90 degrees, 135 degrees, or 165 degrees. The fourth preset angle $\alpha2$ may be adjusted by adjusting the installations position of the emitting element 711 and/or the reflecting element 712. For example, when the installation position of the emitting element 711 is below the installation position of the reflecting element 712, the fourth preset angle $\alpha2$ is less than 90 degrees; when the installation position of the emitting element 711 is upper the installation position of the reflecting element 712, the fourth preset angle $\alpha2$ is larger than 90 degrees. It should be noted that the fourth preset angle $\alpha2$ is not equal to 90 degrees. In some embodiments, in order to have sufficient installation space for the emitting element 711 and the signal feedback component 720, the fourth preset angle $\alpha2$ is less than or equal to 45 degrees.

The signal reflected by the reflecting element 712 can be further projected to the signal feedback component 720 at a fifth preset angle $\lambda2$. The fifth preset angle $\lambda2$ may be larger than 0 and less than 180 degrees. For example, the fifth preset angle $\lambda2$ is 45 degrees, 75 degrees, 90 degrees, 135 degrees, or 165 degrees. The fifth preset angle $\lambda2$ may be adjusted by adjusting the installation positions of the reflecting element 712 and/or the signal feedback component 720.

It should be noted that the signal feedback component 720, the emitting element 711, and the reflecting element 712 may be exemplary embodiments of the signal feedback component 320, the emitting element 311, and the reflecting element 312 illustrated in FIG.3, respectively. The mounting seat 740 may be an exemplary embodiment of the mounting seat 240 illustrated in FIGs. 2A-2B.

It should be noted that the above arrangement manners of the signal feedback component 720, the emitting element 711, and the reflecting element 712 in FIG. 7 are merely provided for illustration purposes, and not intended to limit the scope of the present disclosure. In some embodiments, the installation position of the signal feedback component 720 is below the installation position of the emitting element 711.

In the embodiments of the present disclosure, the reflecting element is provided and arranged on the leaf of the MLC. Compared with arranging the signal feedback component or the signal emitting component on the leaf of the MLC, arranging the reflecting element on the leaf can reduce the safety risk caused by the signal feedback component or the signal emitting component (e.g., wires of the signal feedback component or the signal emitting component) when the leaf moves. In addition, the emitting element is arranged on the mounting seat, which increases the installation space of the emitting element, thereby reducing the limitation on the size of the emitting element.

In some embodiments, the leaves of the MLC are closely arranged along the second direction (i.e., the y-axis direction), each leaf of the MLC corresponds to a position detection device that include a signal feedback component. In order to prevents the signal feedback component from receiving signals corresponding to other leaves of the MLC, a size of the signal feedback component may be smaller than or equal to a size of the corresponding leaf in the second direction. For example, when a size of a leaf of the MLC in the second direction is 1 mm, a size of the signal feedback component corresponding to the leaf less than or equal to 1 mm. In practical applications, in order to improve the accuracy of the radiotherapy, it is often necessary to design the leaves of the MLC to be thinner, for example, the size of the signal feedback component along the second direction may be 0.7 mm.

In some embodiments, to determine the moving stroke of the leaf of the MLC based on the feedback signal, the processor 330 is further configured to determine, based on the feedback signal, a first distance between a target side of the signal feedback component and a current receiving position. The current receiving position refers to a position at the signal feedback component where the signal emitted by the signal emitting component is received at a current time point.

FIG. 8 is a schematic diagram illustrating an exemplary signal feedback component 820 according to some embodiments of the present disclosure. It should be noted that the signal feedback component 820 may be an exemplary embodiment of the signal feedback component 320 illustrated in FIG. 3. As shown in FIG. 8, the signal feedback component 820 is a PIN photoresistor, and the PIN photoresistor includes a P layer, a I layer, and a N layer. When the PIN photoresistor receives the signal emitted by the signal emitting component 310, the P layer is excited and both ends of the PIN photoresistor respectively output current signals (i.e., the feedback signal). The processor 330 may obtain the current signals output from both ends of the PIN photoresistor. Based on the current signals, the processor 330 may determine a first distance L1 between a target side of the PIN photoresistor and a current receiving position P1 where the signal emitted by the signal emitting component 310 is received at the PIN photoresistor at a current time point. For example, the processor 330 may determine the first distance L1 according formula (1) below:

$$\frac{I_1}{I_2} = \frac{R_2}{R_1} = \frac{L_2}{L_1} = \frac{L - L_1}{L_1} \tag{1},$$

where $I_1$ refers to the current value of the current signal output by the target side, $I_2$ refers to the current value of the current signal output by an opposite side of the target side of the PIN photoresistor, $L$ refers to a length of the signal-receiving region on the PIN photoresistor that can receive signals when the leaf moves, $L_2$ refers to a distance between the opposite side of the target side of the PIN photoresistor and the current receiving position P1, $R_1$ refers to a resistance value of a portion of the PIN photoresistor corresponding to the first distance L1, and $R_2$ refers to a resistance value of a portion of the PIN photoresistor corresponding to the distance L2.

Further, to determine the moving stroke of the leaf of the MLC based on the feedback signal, the processor 330 is configured to determine a second distance between the target side of the signal feedback component and a previous receiving position. The previous receiving position refers to a position at the signal feedback component where the signal emitted by the signal emitting component is received at a previous time point prior to the current time point. As shown in FIG. 8, similar to the above process of determining the first distance L1, the processor 330 may determine a second distance L1' between the target side of the PIN photoresistor and a previous receiving position P1'.

Further, the processor 330 is configured to determine the moving stroke of the leaf of the MLC based on the first distance and the second distance. For example, the processor 330 may determine a difference between the first distance L1 and the second distance L1', and designate an absolute value of the difference as the moving stroke of the leaf of the MLC. The moving stroke is the distance that the leaf moves from the previous time point to the current time point.

In some embodiments, a change from the second distance to the first distance is in proportion to or in equal proportion to the moving stroke of the leaf of the MLC. The change from the second distance to the first distance may be a difference between the second distance and the first distance. For example, when signal feedback component is arranged parallel to the moving direction of the leaf of the MLC (for example, as shown in FIG. 6), the change from the second distance to the first distance is in equal proportion to the moving stroke of the leaf of the MLC. As another example, when signal feedback component arranged obliquely to the moving direction of the leaf of the MLC, the change from the second distance to the first distance is in proportion to the moving stroke of the leaf of the MLC. In some embodiments, the processor 330 is configured to determine the moving stroke of the leaf of the MLC based on the relationship between the change from the second distance to the first distance and the moving stroke of the leaf of the MLC.

Further, the processor 330 is configured to determine a current position of the leaf of the MLC based on the moving stroke of the leaf of the MLC. Specifically, the processor 330 may determine the current position of the leaf of the MLC based on the moving stroke and a movement direction of the leaf of the MLC corresponding to the current time point and moving strokes and movement directions of the leaf of the MLC corresponding to all time points prior to the current time point. In some embodiments, the processor 330 may determine the movement direction of the leaf of the MLC corresponding to a time point based on a rotation direction of the driving mechanism of the leaf at the time point. In some embodiments, the processor 330 may determine the movement direction of the leaf of the MLC based on positive and negative properties of the difference between the first distance and the second distance. For example, if the difference is a positive value determined by subtracting L1 from L1', the processor 330 determine that the leaf of the MLC moves towards the target side of the signal feedback component; if the difference is a negative value, the processor 330 determine that the leaf of the MLC moves towards the opposite side of the target side.

The magnetic environment and/or the radiation environment (e.g., the IGRT system) in which the MLC is located may not affect the operation of the components of the position detection device (e.g., the position detection device 300) in the present disclosure, therefore, the position detection device of the MLC in the present disclosure can automatically, accurately, and reliably determine the moving stroke and/or the current position of the leaf of the MLC in the magnetic environment and/or the radiation environment.

In some embodiments, the signal emitting component 310 includes a fiber optic probe, and the signal feedback component 320 includes a detection array. The detection array may include at least two detection profiles extended along the first direction (i.e., the x-axis direction).

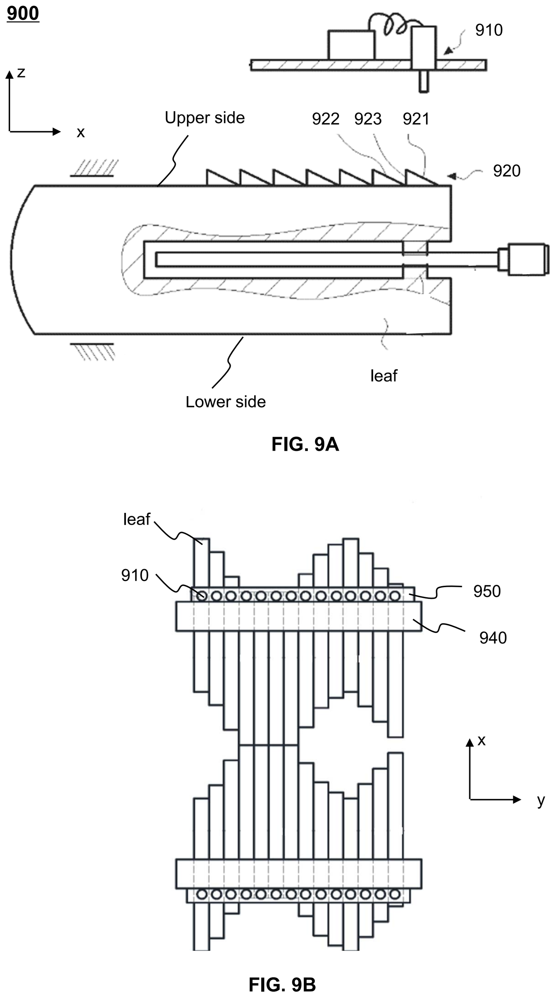

FIG. 9A is a schematic diagram illustrating an exemplary position detection device 900 of a leaf of an MLC according to some embodiments of the present disclosure. It should be noted that the position detection device 900 may be an exemplary embodiment of the position detection device 300 illustrated in FIG. 3. As shown in FIG. 9A, the position detection device 900 may include a fiber optic probe 910, a detection array 920, and a processor (not shown in FIG. 9A). It should be noted that the fiber optic probe 910, the detection array 920, and the processor may be exemplary embodiments of the signal emitting component 310, the signal feedback component 320, and the processor 330 illustrated in FIG. 3.

Conventionally, the fiber optic probe 910 is arranged above the leaf of the MLC along the third direction (i.e., the z-axis direction), and the fiber optic probe 910 is parallel to the first direction, so that an optical signal emitted by the fiber optic probe 910 is horizontally relative to the first direction. However, the space above the leaf where the fiber optic probe 910 can be installed is limited, which limits the size of the fiber optic probe 910, thereby increasing the cost.

In the embodiments of the present disclosure, the fiber optic probe 910 is arranged above or below the leaf of the MLC along the third direction, and the fiber optic probe 910 is perpendicular to the first direction, so that the optical signal emitted by the fiber optic probe 910 is perpendicular to the first direction. For example, as shown in FIG. 9A, the fiber optic probe 910 is arranged above the leaf of the MLC along the third direction and the optical signal emitted by the fiber optic probe 910 is perpendicular to the first direction (that is a detection direction of the fiber optic probe 910 is parallel to the third direction). This arrangement manner requires less installation space for the fiber optic probe 910, thereby reducing the limitation on the size of the fiber optic probe 910, and accordingly reducing the cost.

FIG. 9B is a schematic diagram illustrating exemplary installation positions of fiber optic probes of position detection devices of leaves of an MLC according to some embodiments of the present disclosure. As shown in FIG. 9B, the fiber optic probes 910 of position detection devices corresponding to multiple leaves of the MLC are installed in a mounting seat 940 of a driving mechanism of the multiple leaves of the MLC by a mounting plate 950. By the mounting plate 950, the multiple fiber optic probes 910 can be disassembled and assembled as a whole, which facilitates the disassembly and assembly of the position detection devices.

As shown in FIG. 9B, the multiple fiber optic probes 910 are arranged side by side on the mounting plate 950. In order to make the fiber optic probes 910 have enough installation space, in some embodiments, the multiple fiber optic probes 910 are arranged staggered on the mounting plate 950 along the first direction. In sone alternative embodiment, there are two mounting plate 950 that are respectively mounted at two side of the MLC along the third direction, and a portion of the multiple fiber optic probes 910 are arranged on one mounting plate 950, and the other portion of the multiple fiber optic probes 910 are arranged on the other mounting plate 950.

Correspondingly, the detection array 920 may be arranged on an upper side or a lower side of the leaf of the MLC and move with the leaf. For example, as shown in FIG. 9A, the detection array 920 is arranged on the upper side of the leaf of the MLC.

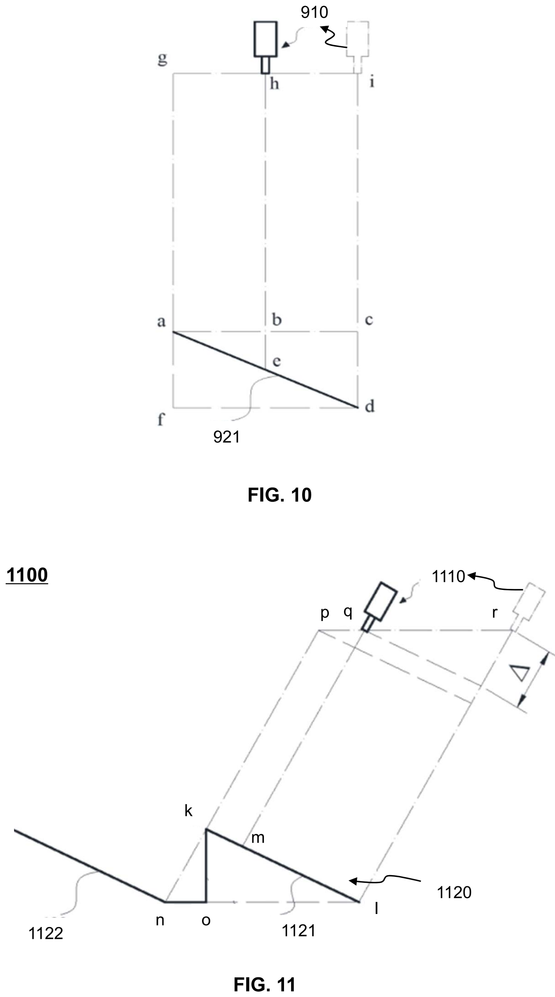

In some embodiments, the fiber optic probe 910 is configured to emit the optical signal to the detection array 920 along the detection direction of the fiber optic probe 910, and the detection array 920 is configured to generate a reflected signal (i.e., the feedback signal) by reflecting the optical signal. The reflected signal may be received by the fiber optic probe 910. The fiber optic probe 910 may generate a processed signal by processing the reflected signal. The processed signal may be used to determine a distance between the fiber optic probe 910 and a position on the detection array 920 where the optical signal is reflected. In some embodiments, the processor is communicatively connected with the fiber optic probe 910 and configured to receive the processed signal from the fiber optic probe 910. For example, the fiber optic probe 910 may convert the processed signal into an electrical signal or other signals (e.g., a digital signal), and the processor may receive the electrical signal or the other signals from the fiber optic probe 910 and obtain the distance from the electrical signal or the other signals. Further, the processor may determine a moving stroke of the leaf of the MLC based on the distance. More descriptions of the determination of the moving stroke of the leaf of the MLC may be found elsewhere in the present disclosure (e.g., FIG. 10 and the descriptions thereof).

In some embodiments, the processor 330 is further configured to determine a current position of the leaf of the MLC based on the moving stroke of the leaf of the MLC.

In some embodiments, the detection array 920 includes at least two detection profiles arranged side by side along the first direction. As shown in FIG. 9A, the detection array 920 includes a first detection profile 921 (denoted by an inclined line) and a second detection profile 922 (denoted by an inclined line). The first detection profile 921 and the second detection profile 922 may be continuous in the first direction. The first detection profile 921 and the second detection profile 922 may be connected by a connection line 923. The first detection profile 921 and the second detection profile 922 may be inclined with respect to the first direction, and the connection line 923 may be perpendicular to the first direction. One detection profile, one connection line, and the upper side of the leaf may form a right triangle. The right angle of the right triangle is the angle between connection line and the first direction. For example, as shown in FIG. 9A, the connection line 923, the first detection profile 921, and the upper side of the leaf form a right triangle. In some embodiments, the detection array 920 and/or the detection profiles may include reflective material. For example, the detection profiles are reflective coating on the detection array 920, and the detection array 920 is metal, plastic, etc. In such cases, the connection line between any two detection profiles is metal, plastic, etc. As another example, the detection array 920 are reflective material. In such cases, the detection profiles and the connection line between any two detection profiles are reflective material.

In some embodiments, the at least two detection profiles of the detection array 920 are fixed on the leaf of the MLC by at least one of screw connection, welding, or riveting. These fixing manners can maintain long-term stability even in the magnetic field environment and the radiation environment (e.g., the IGRT system). In some embodiments, the at least two detection profiles of the detection array 920 have a one-piece structure, and the at least two detection profiles of the detection array 920 are fixed on the leaf of the MLC as a whole. In some embodiments, the at least two detection profiles of the detection array 920 have a separate structure, and each of the at least two detection profiles is respectively fixed on the leaf of the MLC.

In some embodiments, each of the at least two detection profiles includes detection positions capable of being detected by the fiber optic probe 910, and any two detection positions have different distances to the fiber optic probe 910. In some embodiments, all positions on the at least two detection profiles are detection positions.

FIG. 10 is a schematic diagram illustrating an exemplary process of determining a moving stroke of a leaf of an MLC according to some embodiments of the present disclosure. As shown in FIG. 10, at a first time point, the fiber optic probe 910 may detect a detection position $d$ on the first detection profile 921 and determine a distance $id$ (i.e., a detection distance of the fiber optic probe 1110 at the first time point) between the detection position $d$ and the fiber optic probe 910. After the leaf of the MLC moves, at a second time point, the fiber optic probe 910 may detect a detection position e and determine a distance $he$ (i.e., a detection distance of the fiber optic probe 910 at the second time point) between the detection position $e$ on the first detection profile 921 and the fiber optic probe 910. The processor may determine a moving stroke of the leaf between the first time point and the second time point based on the distance $id$ and the distance $he$ according to formula (2) below:

$$bc = fd \times \left(1 - \frac{he-ga}{id-ga}\right) = fd \times \frac{id-he}{fa} \qquad (2),$$

where $bc$ refers to the moving stroke of the leaf between the first time point and the second time point, $fd$ refers to a length of the projection of the first detection profile 921 on the first direction, $fa$ refers to a length of the connection line 923, and $ga$ refers to a detection distance between the fiber optic probe 910 and an end point of the first detection profile 921 far away from the leaf. Further, the processor may determine a moving stroke of the leaf before the second time point according to formula (3) below:

$$vv = fd \times \left(n + \frac{id-he}{fa}\right) \qquad (3),$$

where vv refers to moving stroke of the leaf before the second time point, n refers to the number of detection profiles that the fiber optic probe 910 has passed through.

For each of the at least two detection profiles, the greater an inclination angle of the detection profile relative to the first direction is, the greater the distance difference between the fiber optic probe 910 and any two detection positions on the detection profile is, therefore, the higher the accuracy of the position detection device 900. In some embodiments, the inclination angle of each of the at least two detection profiles is larger than or equal to 30 degrees. In some embodiments, the inclination angle of each of the at least two detection profiles is larger than or equal to 45 degrees. In some embodiments, the inclination angle of each of the at least two detection profiles is larger than or equal to 60 degrees. In some embodiments, the inclination angle of each of the at least two detection profiles is larger than or equal to 60 degrees.

In some embodiments, a total length of the at least two detection profiles along the first direction is not less than a movable distance of the leaf of the MLC, so that the fiber optic probe 910 may detect the signal corresponding to any position of the leaf of the MLC when the leaf of the MLC moves. The movable distance of the leaf refers to a maximum distance that the leaf can move along the first direction.

The position detection device 900 realizes the determination of the moving stroke of the leaf of the MLC in a non-contact manner, so that the detection profiles on the position detection device 900 will not be worn due to use, therefore, the position detection device 900 can maintain high precision during long-term use. In addition, the magnetic environment and/or the radiation environment (e.g., the IGRT system) in which the MLC is located may not affect the operation of the components of the position detection device 900 in the present disclosure, therefore, the position detection device 900 of the MLC in the present disclosure can automatically, accurately, and reliably determine the moving stroke of the leaf of the MLC in the magnetic environment and/or the radiation environment.

FIG. 11 is a schematic diagram illustrating an exemplary position detection device 1100 of a leaf of an MLC according to some embodiments of the present disclosure. It should be noted that the position detection device 1100 may be an exemplary embodiment of the position detection device 300 illustrated in FIG. 3. As shown in FIG. 11, the difference between the position detection device 1100 in FIG. 11 and the position detection device 900 in FIG. 9A is that the optical signal emitted by the fiber optic probe 1110 is inclined at a certain angle (e.g., 60 degrees) relative to the first direction and adjacent detection profiles the detection array 1120 are not continuous in the first direction. It should be noted that an inclination angle of the optical signal emitted by the fiber optic probe 1110 relative to the first direction is the same as an inclination angle of a shortest line connecting any two adjacent detection profiles relative to the first direction, so that the at least two detection profiles of the detection array 1120 is continuous with respect to the detection direction of the fiber optic probe 1110.

In such cases, the processor may determine a moving stroke of the leaf between a third time point and a fourth time point according to formula (4)

$$qr = nl \times \left(\frac{\Delta}{rl-pk}\right) = nl \times \frac{rl-qm}{nk} \qquad (4),$$

where $qr$ refers to the moving stroke of the leaf between the third time point and the fourth time point, $nl$ refers to a length of the projection of a detection profile 1121 on the detection direction of the fiber optic probe 1110, $rl$ refers to a distance (i.e., a detection distance of the fiber optic probe 1110 at the third time point) between a detection position 1 on the detection profile 1121 and the fiber optic probe 1110 at the third time point, $qm$ refers to a distance (i.e., a detection distance of the fiber optic probe 1110 at the fourth time point) between a detection position $m$ on the detection profile 1121 and the fiber optic probe 1110 at the fourth time point, $nk$ refers to a length of a shortest line connecting the detection profile 1121 and the detection profile 1122, and $pk$ refers to a detection distance between the fiber optic probe 1110 and an end point of the first detection profile 1121 far away from the leaf. Further, the processor may determine a moving stroke of the leaf before the fourth time point according to formula (5) below:

$$ww = nl \times \left(n + \frac{rl-qm}{nk}\right) \qquad (5),$$

where $ww$ refers to moving stroke of the leaf before the fourth time point, $n$ refers to the number of detection profiles that the fiber optic probe 1110 has passed through.

In some embodiments, the side of the leaf and a detection profile may from a triangle with a target edge connecting the detection profile and the side of the leaf. For example, the detection profile 1121, the side of the leaf, and the target edge $ko$ form a triangle. The shape of the triangle is changed by changing a position of an intersection between the target edge of the triangle and the side of the leaf. For example, as shown in FIG. 11, the shape of the triangle formed by the detection profile 1121 and the side of the leaf is changed by changing the position of the intersection $o$ of the target edge $ko$ of the triangle. The position of the intersection $o$ may be move to any point between the point $o$ and the point $n$. In some embodiments, the material of the target edge is the same or different from the detection profile. For example, the detection profile is reflective coating, and the target edge is metal, plastic, etc. As another example, the detection profile and the target edge are reflective material.

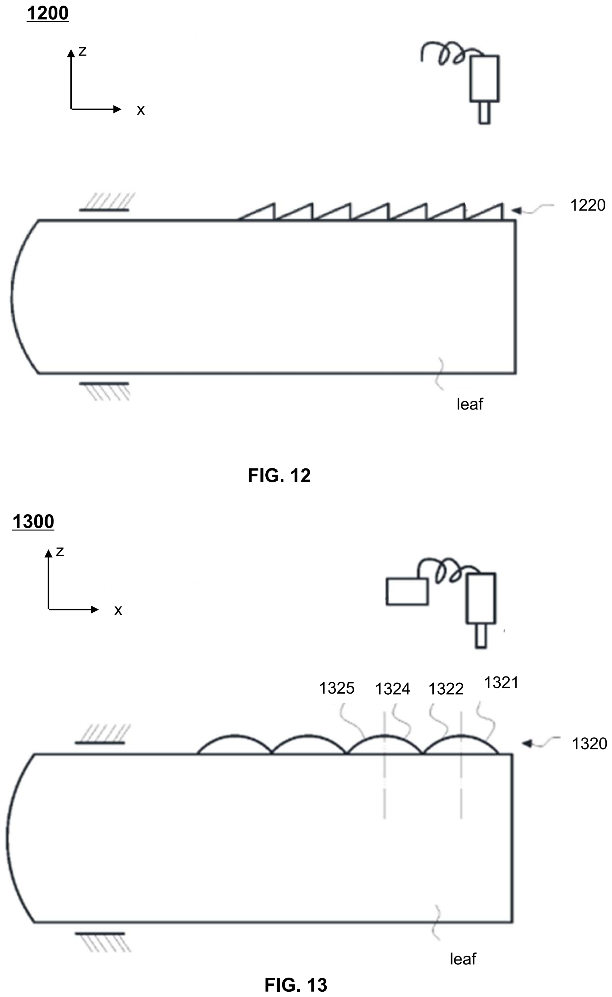

FIG. 12 is a schematic diagram illustrating an exemplary position detection device 1200 of a leaf of an MLC according to some embodiments of the present disclosure. It should be noted that the position detection device 1200 may be an exemplary embodiment of the position detection device 300 illustrated in FIG. 3. As shown in FIG. 12, the difference between the position detection device 1200 in FIG. 12 and the position detection device 900 in FIG. 9A is that inclination directions of the at least two detection profiles of the detection array 1220 are different from the inclination directions of the at least two detection profiles of the detection array 920. In FIG. 9A, the left end of each detection profile is higher than the right end of the detection profile along the third direction, while In FIG. 12, the left end of each detection profile is lower than the right end of the detection profile along the third direction.

FIG. 13 is a schematic diagram illustrating an exemplary position detection device 1300 of a leaf of an MLC according to some embodiments of the present disclosure. It should be noted that the position detection device 1300 may be an exemplary embodiment of the position detection device 300 illustrated in FIG. 3. As shown in FIG. 13, the difference between the position detection device 1300 in FIG. 13 and the position detection device 900 in FIG. 9A is that a shape of each detection profile of the detection array 1320 is different from the shapes of the detection profiles of the detection array 920. The detection array 1320 include multiple detection profile pairs each of which include two symmetrical detection profiles, and the two symmetrical detection profiles can form an arc. For example, as shown in FIG. 13, a detection profile 1321 and a detection profile 1322 are symmetrical and form an arc; a detection profile 1324 and a detection profile 1325 are symmetrical and form an arc. The detection array 1320 with such a structure is convenient to manufacture.

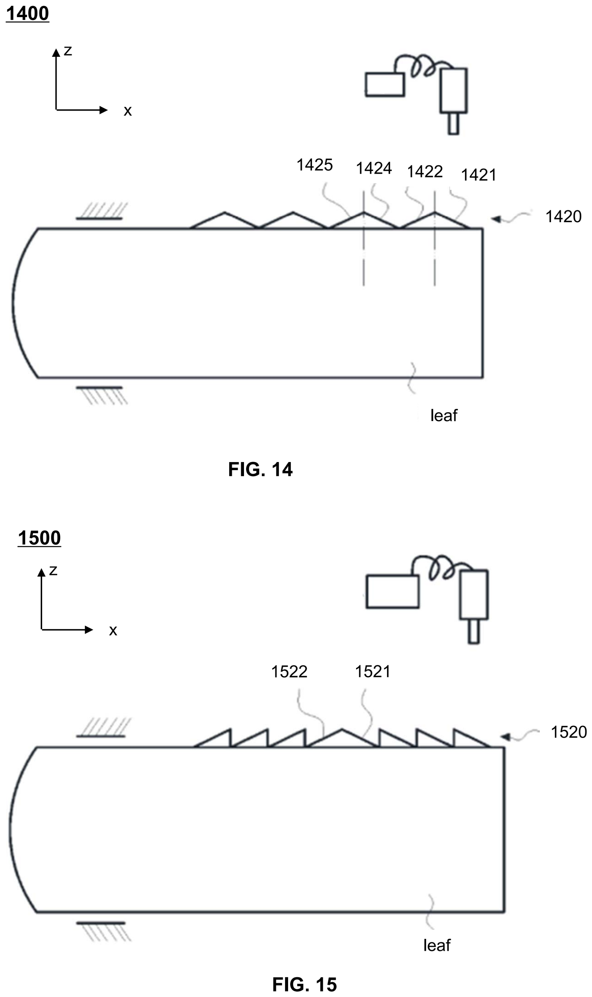

FIG. 14 is a schematic diagram illustrating an exemplary position detection device 1400 of a leaf of an MLC according to some embodiments of the present disclosure. It should be noted that the position detection device 1400 may be an exemplary embodiment of the position detection device 300 illustrated in FIG. 3. As shown in FIG. 14, the difference between the position detection device 1400 in FIG. 14 and the position detection device 900 in FIG. 9A is that a shape of a portion of detection profiles of the detection array 1420 is different from the shapes of detection profiles of the detection array 920. The detection array 1420 include multiple detection profile pairs each of which include two symmetrical detection profiles, and the two symmetrical detection profiles form an isosceles triangle. For example, as shown in FIG. 14, a detection profile 1421 and a detection profile 1422 are symmetrical and form an isosceles triangle; a detection profile 1424 and a detection profile 1425 are symmetrical and form an isosceles triangle. The detection array 1420 with such a structure is convenient to manufacture and assemble.

FIG. 15 is a schematic diagram illustrating an exemplary position detection device 1500 of a leaf of an MLC according to some embodiments of the present disclosure. It should be noted that the position detection device 1500 may be an exemplary embodiment of the position detection device 300 illustrated in FIG. 3. As shown in FIG. 15, the difference between the position detection device 1500 in FIG. 15 and the position detection device 900 in FIG. 9A is that a shape of a portion of detection profiles of the detection array 1520 is different from the shapes of detection profiles of the detection array 920. For example, as shown in FIG. 15, a right portion (e.g., a detection profile 1521) of detection profiles of the detection array 1520 has the same shape as the detection profiles of the detection array 920 in FIG. 9A, and the left portion (e.g., a detection profile 1522) of the detection profiles of the detection array 1520 has the same shape as the detection profiles of the detection array 1220. The left portion of detection profiles of the detection array 1520 are symmetrical to the right portion of detection profiles of the detection array 1520.

It should be noted that the above arrangement manners of the position detection device in FIGs. 9A-9B and FIGs. 11-15 are merely provided for illustration purposes, and not intended to limit the scope of the present disclosure. In some embodiments, the fiber optic probe is arranged below the leaf of the MLC along the third direction, and accordingly, the detection array is arranged on the lower side of the leaf of the MLC.

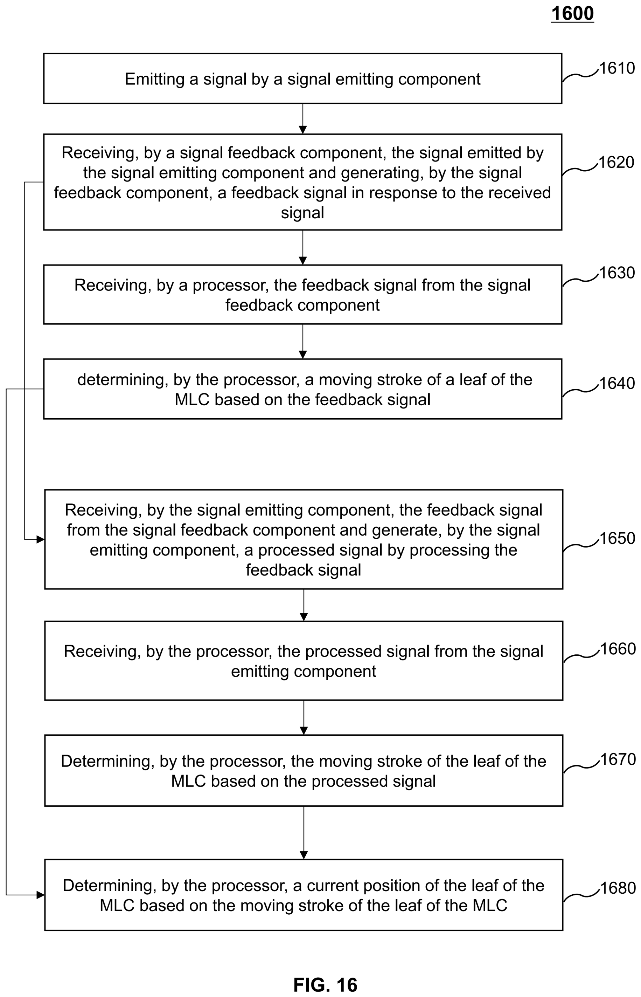

FIG. 16 is a flowchart illustrating an exemplary process for determining a position of a leaf of the MLC according to some embodiments of the present disclosure. For illustration purposes, the implementation of process 1600 by the position detection device 300 is described as an example.

In 1610, a signal emitting component may emit a signal. In some embodiments, the signal emitting component includes an emitting element configured to emit the signal and a reflecting element reflect the signal emitted by the emitting element to a signal feedback component. Merely by way of example, the emitting element is a light-emitting diode or a laser, and the reflecting element is made of reflective material (e.g., a reflector). In some embodiments, the signal emitting component includes a fiber optic probe. More descriptions regarding the signal emitting component may be found elsewhere in the present disclosure (e.g., FIGs. 3-7, FIGs. 9A-9B, FIG. 11, and the description thereof).

In 1620, a signal feedback component may receive the signal emitted by the signal emitting component and generate a feedback signal in response to the received signal. In some embodiments, the signal feedback component includes a PIN photoresistor. In some embodiments, the signal feedback component includes a detection array. More descriptions regarding the signal feedback component may be found elsewhere in the present disclosure (e.g., FIGs. 3-8, FIG. 9A, FIGs. 12-16, and the description thereof).

In 1630, a processor may receive the feedback signal from the signal feedback component. In some embodiments, the processor may be communicatively connected with the signal feedback component.

In 1640, the processor may determine a moving stroke of a leaf of the MLC based on the feedback signal. More descriptions regarding the determination of the moving stroke based on the feedback signal may be found elsewhere in the present disclosure (e.g., FIG. 8 and the description thereof). Further, the processor may perform operation 1680.

In 1650, the signal emitting component may receive the feedback signal from the signal feedback component and generate a processed signal by processing the feedback signal. The processed signal includes a distance between the signal emitting component and a position point on the signal feedback component where the signal emitted by the signal emitting component is received.

In 1660, the processor may receive the processed signal from the signal emitting component. In some embodiments, the processor may be communicatively connected with the signal emitting component.

In 1670, the processor may determine the moving stroke of the leaf of the MLC based on the processed signal. More descriptions regarding the determination of the moving stroke based on the processed signal may be found elsewhere in the present disclosure (e.g., FIG. 10, FIG. 11, and the description thereof). Further, the processor may perform operation 1680.

In 1680, the processor may determine a current position of the leaf of the MLC based on the moving stroke of the leaf of the MLC. More descriptions regarding the determination of the moving stroke based on the processed signal may be found elsewhere in the present disclosure (e.g., FIG. 8 and the description thereof).

The operations of the illustrated process 1600 presented above are intended to be illustrative. In some embodiments, a process may be accomplished with one or more additional operations not described, and/or without one or more of the operations discussed. Additionally, the order in which the operations of a process described above is not intended to be limiting. In some embodiments, the processor may determine the current position of the leaf of the MLC by performing operations 1610-1640 and 1680 or by performing operations 1650-1680.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" may mean that a particular feature, structure or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as a "unit," "module," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2103, Perl, COBOL 2102, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, for example, an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed object matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate $\pm1\%$, $\pm5\%$, $\pm10\%$, or $\pm20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

**Claims**

1.  A position detection device (300) of a multi-leaf collimator (MLC) (114), comprising a fiber optic probe (310), a detection array (320), and a processor (330), wherein:

    the fiber optic probe (310) is arranged above or below a leaf of the MLC (114) along a third direction and configured to emit an optical signal to the detection array (320) along a detection direction of the fiber optic probe (310), the third direction being perpendicular to a first direction and a second direction, the first direction being a moving direction of the leaf of the MLC (114), and the second direction being an arrangement direction of leaves of the MLC (114)

    the detection array (320) is arranged on an upper side or a lower side of the leaf of the MLC (114), moves with the leaf, and configured to generate a reflected signal by reflecting the optical signal,

    the fiber optic probe (310) is further configured to receive the reflected signal and generate a processed signal by processing the reflected signal, and

    the processor (330) is communicatively connected with the fiber optic probe (310) and configured to receive the processed signal from the fiber optic probe (310), obtain, from the processed signal, a distance between the fiber optic probe (310) and a position on the detection array (320) where the optical signal is reflected, and determine a moving stroke of the leaf of the MLC (114) based on the distance.

2.  The position detection device (300) of claim 1, wherein

    the detection array (320) includes at least two detection profiles arranged along the first direction, and
    each of the at least two detection profiles includes detection positions capable of being detected by the fiber optic probe (310), and any two detection positions have different distances to the fiber optic probe (310).

3.  The position detection device (300) of claim 3, wherein the optical signal emitted by the fiber optic probe (310) is perpendicular to the first direction.

4.  The position detection device (300) claim 2 or 3, wherein the detection array (320) includes a first detection profile (921) and a second detection profile (922), the first detection profile (921) and the second detection profile (922) is continuous in the first direction, connected by a connection line (923), and inclined with respect to the first direction, and the connection line (923) is perpendicular to the first direction.

5.  The position detection device (300) of claim 4, wherein one detection profile, one connection line, and the upper side of the leaf form a right triangle, and a right angle of the right triangle is an angle between the connection line and the first direction.

6. The position detection device (300) of claim 5, wherein a left end of each detection profile is higher than a right end of the detection profile along the third direction.

7. The position detection device (300) of claim 5, wherein a left end of each detection profile is lower than a right end of the detection profile along the third direction.

8. The position detection device (300) of claim 5, wherein

in a portion of detection profiles of the detection array (320), a left end of each detection profile is higher than a right end of the detection profile along the third direction, and
in the other portion of the detection profiles of the detection array (320), the left end of each detection profile is lower than the right end of the detection profile along the third direction.

9. The position detection device (300) of any one of claims 2-8, wherein an inclination angle of each of the at least two detection profiles relative to the first direction is larger than or equal to 30 degrees.

10. The position detection device (300) of any one of claims 2-9, wherein a total length of the at least two detection profiles along the first direction is not less than a maximum distance that the leaf is capable of moving along the first direction.

11. The position detection device (300) of claim 2, wherein the optical signal emitted by the fiber optic probe (310) is inclined at a certain angle relative to the first direction, adjacent detection profiles of the detection array (320) are not continuous in the first direction, and an inclination angle of the optical signal emitted by the fiber optic probe (310) relative to the first direction is the same as an inclination angle of a shortest line connecting any two adjacent detection profiles relative to the first direction.

12. The position detection device (300) of claim 2, wherein the detection array (320) includes multiple detection profile pairs each of which includes two symmetrical detection profiles, and the two symmetrical detection profiles can form an arc.

13. The position detection device (300) of claim 2, wherein the detection array (320) includes multiple detection profile pairs each of which includes two symmetrical detection profiles, and the two symmetrical detection profiles form an isosceles triangle.

14. The position detection device (300) of any one of claims 1-13, wherein the fiber optic probe (310) is installed in a mounting seat (940) of a driving mechanism of multiple leaves of the MLC (114) by a mounting plate (950).

15. The position detection device (300) of claim 14, wherein

multiple fiber optic probes (310) corresponding to the multiple leaves of the MLC (114) are arranged side by side on the mounting plate (950), or
the multiple fiber optic probes (310) corresponding to the multiple leaves of the MLC (114) are arranged staggered on the mounting plate (950) along the first direction, or
there are two mounting plate (950) that are respectively mounted at two side of the MLC (114) along the third direction, and a portion of the multiple fiber optic probes (310) corresponding to the multiple leaves of the MLC (114) are arranged on one mounting plate (950), and the other portion of the multiple fiber optic probes (310) are arranged on the other mounting plate (950).

100

112

114

**FIG. 1**

**200**

**FIG. 2A**

**FIG. 2B**

<u>**300**</u>

Signal emitting component

311

Emitting element

312

Reflecting element

310

Signal feedback component

320

Processor

330

**FIG. 3**

**FIG. 4**

**FIG. 5**

**FIG. 6**

**FIG. 7**

**820**

$I_1$                                                 $I_2$

Target side   L1       Signal       L2

L1'       P1       P1'       P-layer

I-layer

N-layer

L

**FIG. 8**

900

910

z

x

Upper side

922  923  921

920

leaf

Lower side

**FIG. 9A**

leaf

910

950

940

x

y

**FIG. 9B**

910

g
h
i

a
b
c

e

f
d

921

**FIG. 10**

**1100**

1110

p q
r

k
m

n o

1122
1121
1120

l

**FIG. 11**

**1200**

**FIG. 12**

**1300**

**FIG. 13**

**1400**

FIG. 14

**1500**

FIG. 15

**1600**

Emitting a signal by a signal emitting component ⟋1610

↓

Receiving, by a signal feedback component, the signal emitted by the signal emitting component and generating, by the signal feedback component, a feedback signal in response to the received signal ⟋1620

↓

Receiving, by a processor, the feedback signal from the signal feedback component ⟋1630

↓

determining, by the processor, a moving stroke of a leaf of the MLC based on the feedback signal ⟋1640

↓

Receiving, by the signal emitting component, the feedback signal from the signal feedback component and generate, by the signal emitting component, a processed signal by processing the feedback signal ⟋1650

↓

Receiving, by the processor, the processed signal from the signal emitting component ⟋1660

↓

Determining, by the processor, the moving stroke of the leaf of the MLC based on the processed signal ⟋1670

↓

Determining, by the processor, a current position of the leaf of the MLC based on the moving stroke of the leaf of the MLC ⟋1680

**FIG. 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202222047797
- CN 202211737667
- CN 202223556585
- CN 202310807984